# EUROPÄISCHE PATENTANMELDUNG

(11) **EP 1 340 486 A1**
(43) Veröffentlichungstag der Anmeldung: **03.09.2003**
(21) Anmeldenummer: 02290508.7
(22) Anmeldetag: 01.03.2002
(51) Int. Cl.: A61K 7/06, A61K 7/16, A61K 7/42, A61K 7/48, A61K 7/50, A61K 31/23, A61P 17/10, A61P 29/00

(54) **Verwendung von Zuckerestern**

(71) Anmelder: Cognis France S.A., 31360 Saint-Martory (FR)
(72) Erfinder: Pauly, Gilles, 54000 Nancy (FR); Freis, Olga, 54280 Seichamps (FR); Danoux, Louis, 54420 Saulxures-Les-Nancy (FR); Gillon, Véronique, 54270 Essey-Les-Nancy (FR); Moussou, Philippe, 54000 Nancy (FR); Grisoni, Philippe, 54760 Bey-Sur-Seille (FR)
(74) Vertreter: Cabinet HERRBURGER

(57) **Zusammenfassung**

Vorgeschlagen wird die Verwendung von Zuckerestern als Wirkstoffe zur Herstellung von kosmetischen und/oder pharmazeutischen Zubereitungen.

## Beschreibung

### Gebiet der Erfindung

Die Erfindung befindet sich auf dem Gebiet der Kosmetik und betrifft die Verwendung von Zuckerestern als Wirkstoffe für die Herstellung von Haut- und Haarpflegemitteln.

### Stand der Technik

Für die Herstellung von kosmetischen Zubereitungen, speziell Hautpflegemittel werden Wirkstoffe benötigt, die sich über ein komplexes Anforderungsprofil auszeichnen : sie sollen speziell der Haut, aber auch den Haaren Schutz gegen über Umweltgiften, oxidativen Stress und UV-Strahlung verleihen, der Faltenbildung vorbeugen, anti-inflammatorisch wirksam, aber gleichzeitig auch besonders hautmild sein. Da mit der Entwicklung und Zulassung solcher "kosmetischen Vielzweckwaffen" stets ein hoher Kosten- und Zeitaufwand verbunden ist, besteht ein besonderes Interesse an solchen Wirkstoffen, die bereits für den Einsatz in Kosmetik und Pharmazie bekannt und zugelassen sind, deren Wirkstoffpotential bisher jedoch nicht bekannt oder nicht hinreichend erforscht ist.

Seit vielen Jahren haben nichtionische Tenside vom Typ der Glycosidfettsäureester, gemeinhin als Zuckerester bezeichnet, wegen ihrer besonderen Hautverträglichkeit, biologischen Abbaubarkeit und hohen Emulgierfähigkeit besondere Bedeutung für zahlreiche Anwendungen gefunden, wie beispielsweise die Herstellung von Emulsionen für die Kosmetik, Körperpflegemittel, Shampoos, Haarsprays, Zahnpasten, Lippstiften, Mascaras und dergleichen. Besonders umfangreich sind Herstellung und Anwendung von Fettsäureestern der Sucrose, der Methylglucose, der Fructose und der Trehalose beschrieben. Ihre besondere Milde, ihr Beitrag zur Feuchtigkeitsregulierung der Haut sowie ihre Verwendung zur Verringerung des Irritationspotentials von Aniontensiden oder AHA wird beispielsweise von Desai in **Cosm.Toil. 105, p99-107 (1990)** beschrieben.

In diesem Zusammenhang sei auf die französischen Patentanmeldung **FR-A1 9211770** (L'Oreal) verwiesen, aus der die Verwendung von Fructoseoctanoat zur Wiederherstellung des Lipidfilms auf der Haut und zur Verhinderung des transepidermalen Wasserverlustes (TEWL) entfetteter Haut bekannt ist. In den Druckschriften **JP-A1 03/261711** und **JP 03/197414** werden Dextroseester für die Verbesserung von Weichgriff, Kämmbarkeit und Feuchtigkeit von Haaren vorgeschlagen. Aus dem japanischen Patent **JP-A1 09/241404** (Lion) ist der Einsatz von Estern der Glucose mit C6-C9-Fettsäuren gegen gram-positive Bakterien und ihre Verwendung zur Herstellung von bakteriziden Zubereitungen bekannt. In den beiden Patentanmeldungen EP-A1 0875239 und EP-A1 0985408 (BDF) wird der Einsatz von Estern von Fettsäuren mit Di- oder Oligosacchariden gegen die Haftung von Mikroorganismen auf harten Oberflächen vorgeschlagen.

Die Aufgabe der vorliegenden Erfindung hat demnach darin bestanden, solche bekannten kosmetischen Einsatzstoffe zu identifizieren, die sich über ihre geläufigen Eigenschaften durch ein komplexes Wirkstoffprofil auszeichnen. Insbesondere sollten diese Stoffe das Zellpotential verbessern, um die Mitochondrien zur erhöhten Produktion von Energie zu veranlassen, die beispielsweise benötigt wird, um Haut und Haare gegen oxidativen Stress und Umweltgifte zu schützen. Gleichzeitig sollten die Stoffe anti-inflammatorisch und speziell gegen solche Keime wirksam sein, die in die Entstehung von *Acne* vulgarisinvolviert sind.

### Beschreibung der Erfindung

Gegenstand der Erfindung ist die Verwendung von Zuckerestern als Wirkstoffe zur Herstellung von kosmetischen und/oder pharmazeutischen Zubereitungen.

Überraschenderweise wurde gefunden, dass Zuckerester, die schon seit vielen Jahren als Emulgatoren für Nahrungsmittel, Kosmetika und pharmazeutische Zubereitungen bekannt und im Handel sind, schon in kleinsten Konzentrationen über ein umfangreiches Profil als Wirkstoff verfügen. Weitere Gegenstände der Erfindung betreffen daher ihre Verwendung
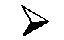 als anti-inflammatorische Wirkstoffe,
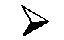 als Wirkstoffe zum Schutz von Haut und Haaren gegen die Einwirkung von UV-A- und UV-B-Strahlen,
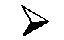 als Wirkstoffe zum Schutz von Haut und Haaren gegen Umweltgifte und oxidativen Stress,
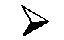 als Anti-Ageingmittel,
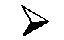 als Anti-Protease-Wirkstoffe, speziell als Anti-Collagenase-Wirkstoffe,
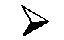 als Wirkstoffe zur Inhibierung der Melaninsynthese in Haar- und Hautzellen und somit als Hautweißungsmittel sowie
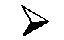 als Anti-Akne-Wirkstoffe.

### Zuckerester

Zuckerester stellen bekannte nichtionische Tenside dar, die nach den einschlägigen Methoden der präparativen organischen Chemie erhältlich sind, beispielsweise durch Umesterung von Fettsäuremethylestern mit den entsprechenden Zuckern oder auf enzymatischem Wege, wie dies beispielsweise in der internationalen Patentanmeldung **WO 99/02722** (Laboratoires Serobiologiques) beschrieben wird. Zuckerester mit unterschiedlichsten Glycosid- und Acylkomponenten sind im Handel erhältlich, beispielsweise von den Firmen Sisterna oder Ryoto. Typische Beispiele für geeignete Zuckerester sind in der folgenden Abbildung dargestellt: 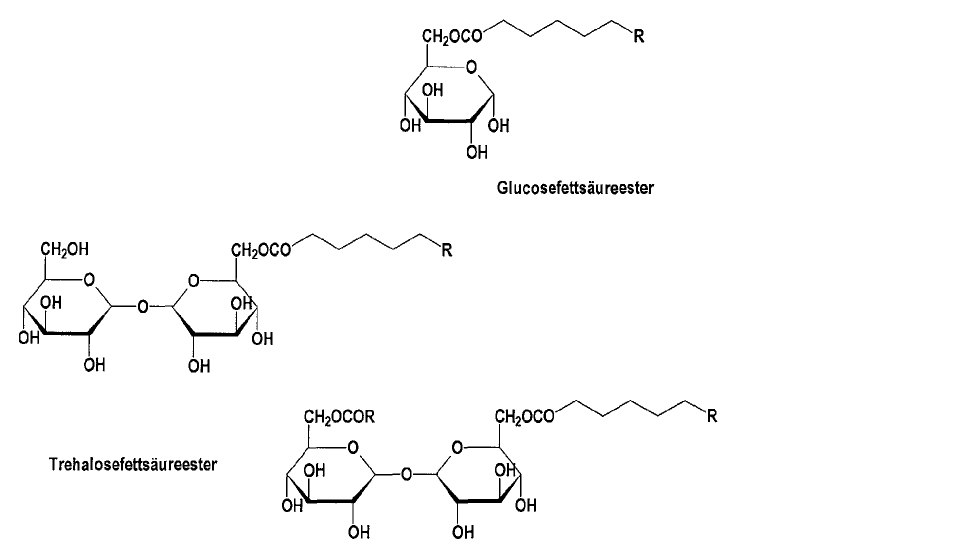 Grundsätzlich kommen Zuckerester in Frage, die sich von Mono-, Di und/oder Oligosacchariden ableiten. Dabei kann es sich um Aldohexosen (z.B. Glucose, Methylglucose, Mannose, Galactose); Deoxyaldosen (z.B. Rhamnose, Fucose, Deoxyribose); Aldopentosen (z.B. Ribose, Arabinose, Xylose); Ketosen (z.B. Fructose in Pyranosyl- oder Furanosylform); Disaccharide (z.B. Trehalose, Sucrose, Maltose, Isomaltose, Cellobiose, Melibiose, Gentobiose, Lactose) sowie Tri-, Tetra-, Penta- und Oligosaccharide handeln. Vorzugsweise kommen Fructose- Glucose-, Trehalose- und/oder Sucroseester zum Einsatz. Die Acylkomponente der Ester kann sich von Fettsäuren der Formel (I) ableiten,

R¹CO-OH (I)

in der R¹CO für einen linearen oder verzweigten, gesättigten oder ungesättigten Acyl- oder Hydroxyacylest mit 6 bis 22, vorzugsweise 8 bis 16 Kohlenstoffatomen und 0 und/oder 1 bis 3 Doppelbindungen steht. Typische Beispiele sind Zuckerester der Capronsäure, 2-Hydroxycapronsäure, 6-Hydroxycapronsäure, Caprylsäure, 2-Ethylhexansäure, Caprinsäure, 10-Hydroxycaprinsäure, Laurinsäure, 12-Hydroxylaurinsäure, Isotridecansäure, Myristinsäure, Palmitinsäure, Palmoleinsäure, 16-Hydroxypalmitinsäure, Stearinsäure, Isostearinsäure, Ölsäure, Elaidinsäure, Petroselinsäure, Linolsäure, Linolensäure, Elaeostearinsäure, 12-Hydroxystearinsäure, Ricinolsäure, Arachinsäure, Gadoleinsäure, Behensäure und Erucasäure sowie deren technische Mischungen. Ferner können sich die Zuckerester auch von Dicarbonsäuren mit 2 bis 22, vorzugsweise 6 bis 18 Kohlenstoffatomen ableiten, wie beispielsweise Adipinsäure oder Hexadecandicarbonsäure. Die Ester können je nach zur Verfügung stehender Hydroxylgruppen 1 bis 8 Estergruppen aufweisen; vorzugsweise werden jedoch solche Produkte eingesetzt, die einen Veresterungsgrad von durchschnittlich 1 bis 6 und insbesondere 1,5 bis 2,5 aufweisen.

### Kosmetische und/oder pharmazeutische Zubereitungen

Die Zuckerester werden erfindungsgemäß zur Herstellung von kosmetischen und/oder pharmazeutischen Zubereitungen wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben verwendet, in denen sie in Mengen von 0,0001 bis 10, vorzugsweise 0,001 bis 5 und insbesondere 0,01 bis 1 Gew.-% - bezogen auf die Mittel - enthalten sind. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
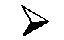 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
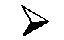 Alkyl- und/oder Alkenyloligoglykoside mit 8 bis 22 Kohlenstoffatomen im Alk(en)ylrest und deren ethoxylierte Analoga;
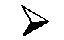 Anlagerungsprodukte von 1 bis 15 Mol Ethylenoxid an Ricinusöl und/oder gehärtetes Ricinusöl;
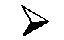 Anlagerungsprodukte von 15 bis 60 Mol Ethylenoxid an Ricinusöl und/oder gehärtetes Ricinusöl;
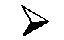 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
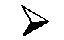 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
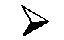 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
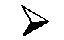 Mono-, Di- und Trialkylphosphate sowie Mono-, Di- und/oder Tri-PEG-alkylphosphate und deren Salze;
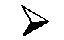 Wollwachsalkohole;
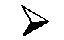 Polysiloxan-Polyalkyl-Polyether-Copolymere bzw. entsprechende Derivate;
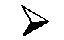 Block-Copolymere z.B. Polyethylenglycol-30 Dipolyhydroxystearate;
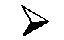 Polymeremulgatoren, z.B. Pemulen-Typen (TR-1,TR-2) von Goodrich;
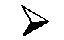 Polyalkylenglycole sowie
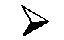 Glycerincarbonat.

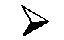 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

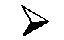 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

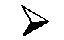 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

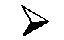 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

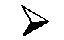 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

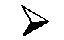 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

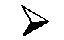 Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl-oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinyl pyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil. 108, 95 (1993) aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976)**.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
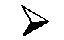 3-Benzylidencampher bzw. 3-Benzylidennorcampher und dessen Derivate, z.B. 3-(4-Methylbenzyliden)campher wie in der **EP 0693471 B1** beschrieben;
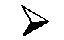 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
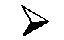 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
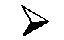 Ester der Salicylsäure, vorzugsweise Salicylsäure-2-ethylhexylester, Salicylsäure-4-isopropylbenzylester, Salicylsäurehomomenthylester;
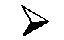 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
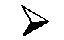 Ester der Benzalmalonsäure, vorzugsweise 4-Methoxybenzmalonsäuredi-2-ethylhexylester;
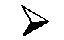 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
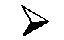 Propan-1,3-dione, wie z.B. 1-(4-tert.Butylphenyl)-3-(4'methoxyphenyl)propan-1,3-dion;
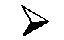 Ketotricyclo(5.2.1.0)decan-Derivate, wie in der **EP 0694521 B1** beschrieben.

Als wasserlösliche Substanzen kommen in Frage:
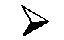 2-Phenylbenzimidazol-5-sulfonsäure und deren Alkali-, Erdalkali-, Ammonium-, Alkylammonium-, Alkanolammonium- und Glucammoniumsalze;
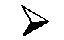 Sulfonsäurederivate von Benzophenonen, vorzugsweise 2-Hydroxy-4-methoxybenzophenon-5-sulfonsäure und ihre Salze;
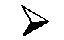 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122,543 (1996) sowie Parf.Kosm. 3, 11 (1999) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmendeu Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

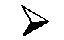 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-ndecylamid.

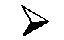 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterinund Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

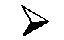 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

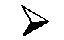 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
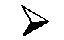 adstringierende Wirkstoffe,
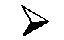 Ölkomponenten,
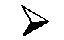 nichtionische Emulgatoren,
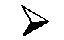 Coemulgatoren,
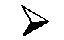 Konsistenzgeber,
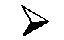 Hilfsstoffe wie z. B. Verdicker oder Komplexierungsmittel und/oder
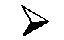 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
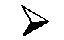 entzündungshemmende, hautschützende oder wohlriechende ätherische Öle,
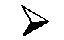 synthetische hautschützende Wirkstoffe und/oder
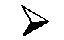 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(lH-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion /Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
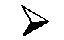 Glycerin;
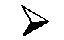 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
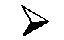 technische Oligoglyceringemische mit einem Eigenkondensationsgrad von 1,5 bis 10 wie etwa technische Diglyceringemische mit einem Diglyceringehalt von 40 bis 50 Gew.-%;
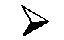 Methyolverbindungen, wie insbesondere Trimethylolethan, Trimethylolpropan, Trimethylolbutan, Pentaerythrit und Dipentaerythrit;
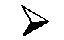 Niedrigalkylglucoside, insbesondere solche mit 1 bis 8 Kohlenstoffen im Alkylrest, wie beispielsweise Methyl- und Butylglucosid;
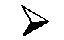 Zuckeralkohole mit 5 bis 12 Kohlenstoffatomen, wie beispielsweise Sorbit oder Mannit,
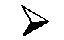 Zucker mit 5 bis 12 Kohlenstoffatomen, wie beispielsweise Glucose oder Saccharose;
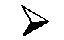 Aminozucker, wie beispielsweise Glucamin;
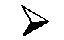 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

In den nachfolgenden Beispielen wurden enzymatisch hergestellte Zuckerester der PRABIL (Plateforme Regionale AgroBioindustrielle de Lorraine) eingesetzt, welche gemäß der Vorschrift der Internationalen Patentanmeldung WO 99/02722 (Laboratoire Sérobiologiques) hergestellt worden waren. Die Reinigung erfolgte durch Flüssig/Flüssig-Extraktion oder Extraktion mit überkritischem Kohlendioxid. Bei den eingesetzten Sucroseestern (SE) handelte es sich um Verkaufsprodukte der Firmen Sisterna und Ryoto. Die genaue Zusammensetzung der Ester ist in Tabelle 1 wiedergegeben:

**Tabelle 1:**

| Zusammensetzung der eingesetzten Zuckerester | | | | |
|---|---|---|---|---|
| Bez. | Zuckerester | Mono/Diester-Verhältnis | Restgehalt Zucker Gew.-% | Restgehalt Fettsäure Gew.-% |
| FC | Fructosecaprat | 58 :42 | 5 | < 5 |
| FP | Fructosepalmitat | 49 : 51 | < 3 | < 5 |
| FS | Fructosestearat | 49 : 51 | < 3 | < 3 |
| GC | Glucosecaprylat | > 98 : < 2 | < 5 | 11 |
| GL | Glucoselaurat | > 98 : < 2 | < 5 | 8 |
| GP | Glucosepalmitat | > 98 : < 2 | < 5 | < 5 |
| TC | Trehalosecaprat | 57 : 43 | < 5 | < 5 |
| TL | Trehaloselaurat | 33 : 77 | < 5 | < 5 |
| TP | Trehalosepalmitat | 52 : 48 | < 5 | < 5 |
| TS | Trehalosestearat | 48 : 52 | < 5 | < 5 |

### A) Regenerative und wachstumsstimulierende Wirkung

Nach einer Inkubation von 72 h in einer Nährlösung bilden Fibroblasten gesättigte Monolayer aus, die Fibroblasten stellen ihre Aktivität ein und das Wachstum kommt zum Stillstand. Der Zellbrennstoff Adenosintriphosphat (ATP), der im wesentlichen in den Mitochondrien entsteht, wird zur Aktivierung von bestimmten Enzymen benötigt, die beispielsweise das Zellskelett kontrollieren, die lonenkanäle, die Aufnahme von Nährstoffe und eine ganze Reihe anderer wichtiger biologischer Abläufe. Die Bestimmung des Proteingehaltes in den Zellen erfolgte nach der Methode von Bradford [ vgl. Anal. Biochem. **72, 248-254 (1977)** ]. Glutathion (GSH) ist ein spezielles Protein, welches von den Zellen zum Schutz gegen oxidativen Stress und Umweltgifte, insbesondere gegen Schwermetalle produziert wird. Die an der reduzierten Form des GSH beteiligten drei Aminosäuren sind mit speziellen cytoplasmatischen Enzymen verknüpft, die zur Aktivierung ATP benötigen. Ein Anstieg der GSH-Konzentration führt zu einem Anstieg der Glutathion-S-transferase-Aktivität, eines Entgiftungsenzyms. Der GHS-Gehalt wurde nach der Methode von Hissin [vgl. Anal. Biochem. **74,214-226 (1977)**] bestimmt. Die *wachstumsstimulierende Wirkung* der Testsubstanzen wurden an menschlichen Fibroblasten untersucht. Dazu wurden in einer ersten Testreihe die Fibroblasten in einem Nährmedium 1 Tag bei 37 °C und 5 Vol.-% CO₂ inkubiert, das Nährmedium gegen ein Medium, welches die Testsubstanzen enthielt, ausgewechselt und wiederum 3 Tage bei 37 °C inkubiert. Anschließend wurde die Mitochondrienaktivität gemäß dem MTT-Test (Denizot, 1986) bestimmt; hierzu wurde MTT mit Hilfe eines Enzyms aus der Atmungskette der Succinat Dehydrogenase in Formazan überführt. Zur Bestimmung der *überlebensstimulierenden Wirkung* wurden in einer zweiten Testreihe die Fibroblasten zunächst 3 Tage bei 37 °C in einer Nährlösung und dann 3 Tage bei gleicher Temperatur in einer Testlösung inkubiert. Anschließend wurde der Proteingehalt in den Zellen sowie die GSH-Konzentration bestimmt. Die Ergebnisse sind in Tabelle 2 zusammengefasst. Angege-ben sind die Ergebnisse von jeweils 3 Messreihen mit Dreifachbestimmung in %-rel gegen-über einer Blindprobe.

**Tabelle 2**

| Wachstums- und überlebensstimulierende Wirkung (Angabe in %-rel.) | | | | |
|---|---|---|---|---|
| Zuckerester | Konz. % w/v | MTT | Proteine | GSH / proteine |
| Blindprobe | 0 | 100 | 100 | 100 |
| Trehalosepalmitat | 0,001 | 112 | 100 | 125 |
| Trehalosestearat | 0,001 | 116 | 94 | 141 |
| Sucrosestearat* | 0,003 | 131 | 123 | 170 |

| | | | | |
|---|---|---|---|---|
| *) Sisterna SP50C | | | | |

Die Beispiele zeigen, dass die Testsubstanzen den Metabolismus im Hinblick auf das Wachstum und den Schutz der Fibroblasten stimulieren.

### B) Anti-stress Wirkung gegenüber UV-A-geschädigten Fibroblasten

UV-A-Strahlung im Bereich von 320 bis 400 nm induziert einen oxidativen Stress der hauptsächlich durch die Aktivierung von photosensitiven biologischen Komponenten hervorgerufen wird, welche ihrerseits die Bildung von ROS-ähnlichen Superoxidanionen, Wasserstoffperoxid und Singulettsauerstoffatomen katalysieren. Die Anti-UV-A-Wirkung wurde an Fibroblasten untersucht, da die UV-A-Strahlung durch die Dermis tritt, wo sie eine oxidative Schädigung durch Lipoperoxidation der Zellmembran sowie einer Abnahme des Gehaltes an reduzierten Glutathions (GHS) bewirkt. In diesem Sinne wurden menschliche Fibroblasten wie unter A) bereits beschrieben kultiviert, mit UV-A-Strahlen (20 J/Cm2) behandelt und dann die Zellzahlen sowie der GSH-Gehalt bestimmt. Die Ergebnisse sind in Tabelle 3 zusammengefasst. Angegeben sind die Ergebnisse von jeweils 3 Messreihen mit Dreifachbestimmung in %-rel gegenüber einer Blindprobe.

**Tabelle 3**

| Anti-Stresswirkung (Angabe in %-rel.) | | | |
|---|---|---|---|
| Zuckerester | Konz. % w/v | Zellproteine | GSH/Proteine |
| Blindprobe | 0 | 100 | 100 |
| Blindprobe + UV-A | 0 | 105 | 65 |
| Vitamin E + UV-A | 0,0003 | 111 | 81 |
| Fructosepalmitat + UV-A | 0,003 | 91 | 81 |
| Fructosestearat + UV-A | 0,003 | 83 | 79 |
| Glucosecaprat + UV-A | 0,003 | 109 | 144 |
| Glucoselaurat + UV-A | 0,0001 | 95 | 91 |
| Glucosepalmitat + UV-A | 0,003 | 119 | 114 |
| Trehalosecaprat + UV-A | 0,00003 | 120 | 87 |
| Trehaloselaurat + UV-A | 0,0003 | 119 | 90 |
| Trehalosepalmitat + UV-A | 0,001 | 124 | 87 |
| Trehalosestearat + UV-A | 0,001 | 124 | 84 |

Durch den Einsatz der Zuckerester wurde der Gehalt an GSH in den Fibroblasten gegenüber der UV-A-Einstrahlung geschützt.

### C) Zellschutz gegenüber UV-B-Strahlen

Aufgabe dieses Tests war es zu zeigen, dass die Testsubstanzen anti-inflammatorische Eigenschaften gegenüber menschlichen Keratinocyten besitzen. UVB wurde als Stressfaktor ausgewählt, weil die Strahlen durch Aktivierung von Arachidonsäure freisetzenden Enzymen, wie beispielsweise Phospholipase A2 (PLA2) eine cutane Inflammation (Erytheme, Ödeme) hervorrufen. Dies führt in der Folge nicht nur zu einer Schädigung der Membranen, sondern auch zur Bildung von inflammatorisch wirkenden Stoffen, wie beispielsweise Prostaglandinen vom Typ PGE2. Der Einfluss der UVB-Strahlen auf die Keratinocyten wurde in-vitro über die Freisetzung von cytoplasmatischen Enzymen, wie z.B. LDH (Lactat Dehydrogenase) bestimmt, die parallel zur Zellschädigung und der Bildung von PGE2 verläuft. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVB-Strahlungsmenge geschädigt (50 mJ/cm²). Die Keratinocytenmenge wurde nach Trypsination über einen Zellzähler ermittelt, die LDH-Konzentration enzymatisch bestimmt. Die Ergebnisse sind in Tabelle 4 zusammengefasst. Angegeben ist die Aktivität in %-rel gegen einen Standard als Mittelwert von zwei Testreihen mit Doppelbestimmung.

**Tabelle 4**

| Wirkung gegen UVB-Strahlen (Angabe in %-rel.) | | | | |
|---|---|---|---|---|
| Zuckerester | Konz. % w/v | Cellulare DNA | Freigesetztes LDH | Freigesetztes PGE2 |
| Blindprobe | 0 | 100 | 0 | 0 |
| Blindprobe + UV-B | 0 | 33 | 100 | 100 |
| Fructosecaprat + UV-B | 0,001 | 32 | 68 | 35 |
| | 0,003 | 140 | 0 | 0 |
| Glucosecaprat + UV-B | 0,003 | 28 | 72 | 50 |
| | 0,01 | 36 | 48 | 28 |
| Glucoselaurat + UV-B | 0,001 | 17 | 105 | 107 |
| | 0,003 | 54 | 49 | 23 |
| Glucosepalmitat + UV-B | 0,001 | 32 | 68 | 63 |
| | 0,003 | 32 | 68 | 65 |

Die Ergebnisse zeigen, dass die Testsubstanzen die schädigenden Einflüsse von UVB-Strahlen signifikant reduzieren und insbesondere die Freisetzung von LDH und PGE2 vermindern.

### D) Anti-inflammatorische Wirkung

Im Verlauf einer cutanen Inflammation werden Leucocyten, wie beispielsweise die polymorphonuclearen neutrophilen Granulocyten (PMN) durch Peptide wie etwa Cytokine stimuliert, Botschafterstoffe wie z.B. Leukotrien auszusenden, die von aktivierten oder nekrotischen Zellen in der Dermis freigesetzt werden. Diese aktivierte PMN setzen nicht nur pro-inflammatorische Cytokine, Leukotriene und Proteasen, sondern auch ROS, wie z.B. Superoxide und Hypochloritanionen frei, welche die Aufgabe haben, eingedrungene pathogene Keime oder Pilze zu vernichten. Diese Aktivität der PMN während der Inflammation ist als sogenannter Atmungsausbruch ("respiratory burst") bekannt. Zur Untersuchung, inwiefern die Testsubstanzen den Atmungsausbruch verhindern oder mindern können, wurde eine Zelllinie von menschlichen leukemischen Granulocyten dieser PMN zusammen mit den Testsubstanzen bei 37 °C von 5 Vol.-% CO₂ inkubiert. Nach Auslösung des Atmungsausbruchs durch Zugabe eines Hefeextraktes (Zymosan) zur Zelllösung, wurde die Freisetzung von Superoxidanionen über deren Reaktion mit Luminol bestimmt. Die Ergebnisse sind in Tabelle 5 zusammengefasst. Angegeben sind die Zellzahlen sowie die Menge an freigesetzten ROS in %-rel zum Standard als Mittelwert einer Messreihe mit Dreifachbestimmung.

**Tabelle 5**

| Anti-inflammatorische Wirkung (Angabe in %-rel.) | | | |
|---|---|---|---|
| Zuckerester | Konz. % w/v | Zellzahlen | Freigesetzte ROS |
| Blindprobe + Stimulation | 0 | 100 | 100 |
| Fructosecaprat + Stimulation | 0,003 | 97 | 87 |
| | 0,01 | 60 | 5 |
| Fructosepalmitat + Stimulation | 0,001 | 97 | 81 |
| | 0,01 | 98 | 42 |
| Fructosestearat + Stimulation | 0,001 | 100 | 88 |
| | 0,01 | 155 | 26 |
| Glucosepalmitat + Stimulation | 0,001 | 102 | 99 |
| | 0,01 | 91 | 58 |

Die Ergebnisse zeigen, dass die Testsubstanzen einen starken inhibierenden Einfluss auf den Atmungsausbruch menschlicher Granulocyten besitzen, ohne die Granulocyten zu schädigen.

### E) Wirksamkeit gegen Proteasen

Während einer Inflammation, werden aus den polymorphonuclearen neutrophilen Granulocyten oder Makrophagen Hautproteasen, wie beispielsweise Collagenase freigesetzt. Ein ähnlicher Vorgang spielt sich gerade in der Haut älterer Menschen bei Einfluss von UV-Strahlen ab. Die Proteasen - wegen ihres Gehaltes an zentralen Zinkionen auch als Matrix-Metallo-Proteasen (MMP) bezeichnet - katalysieren wie schon erwähnt die Fragmentierung von Bindegewebsproteinen. Zur Untersuchung der Testsubstanzen auf Collagenaseinhibierung wurde bakterielle Collagenase (clostridium histolyticum) auf Gelatine als natürlichem Nährboden verwendet, welche mit Fluorochrom (FITC, Calbiochem) markiert war. Die Inkubationszeit betrug 60 min bei 20 °C, die Hydrolyse des Substrates wurde über die Fluoreszenz Dei 393 nm (Anregung bei 328 nm) verfolgt. Die Ergebnisse sind in Tabelle 6 zusammengefasst. Angegeben ist die Collagenase-Inhibierung in %.

**Tabelle 6**

| Collagenase-Inhibierung (Angabe in %-rel.) | | |
|---|---|---|
| Zuckerester | Konz. [w/v] | Collagenase-Inhibierung |
| Blindprobe | 0 | 0 |
| Fructosepalmitat | 0,03 | 4 |
| | 0,1 | 41 |
| | 0,3 | 78 |
| Fructosestearat | 0,3 | 45 |
| Glucoselaurat | 0,1 | 28 |
| Trehalosecaprat | 0,3 | 52 |
| Trehaloselaurat | 0,1 | 21 |
| Trehalosepalmitat | 0,3 | 9 |

Die Ergebnisse zeigen, dass die Testsubstanzen in Abhängigkeit der Konzentration über eine signifikante Inhibierungswirkung verfügen.

### F) Inhibierung der Melaninsynthese in B16-Melanocyten

Melanin ist für die Pigmentierung von Haut und Haaren verantwortlich. Die Synthese dieses Pigmentes findet in speziellen Organellen, den sogenannten Melanosomen statt, welche in den Melanocyten der Basalschicht der menschlichen Epidermis zu finden sind. Die Biosynthese geht von der Aminosäure Tyrosin aus, die in Gegenwart von Tyrosinase zu DOPA (Dihydroxyphenylalanin) oxidiert wird, welches dann seinerseits zu Melanin polymerisiert. Zum Nachweis der Inhibierung der Melanininhibierung wurden Melanocyten (B16 Zelllinie) in einem Standardmedium inoculiert. Nach einer Inkubationszeit von 3 Tagen bei 37 °C und 5 Vol.-% CO₂ wurde das Nährmedium gegen eine Lösung gewechselt, welche die Testsubstanzen in unterschiedlichen Konzentrationen enthielt. Nach einer erneuten Inkubationszeit von 3 Tagen wurde der Gehalt an Zellproteinen (Bradford-Methode) und gebildetem Melanin über die optische Dichte des Homogenisates bei 475 nm bestimmt. Die Ergebnisse sind angegeben in %-rel gegenüber einem Blindwert und in Tabelle 7 zusammengefasst.

**Tabelle 7:**

| Melaninsynthese in B16 Melanocyten [%-rel.] | | | |
|---|---|---|---|
| Zuckerester | Konz. w/v. | Zellproteine | Zellmelanin |
| Blindwert | 0 | 100 | 100 |
| Fructosecaprat | 0,003 | 108 | 54 |
| | 0,006 | 80 | 30 |
| Fructosestearat | 0,006 | 105 | 49 |
| | 0,01 | 76 | 21 |
| Trehalosecaprat | 0,001 | 100 | 53 |
| | 0,003 | 75 | 23 |
| Trehalosestearat | 0,001 | 97 | 81 |
| | 0,003 | 93 | 41 |
| Sucrosestearat ¹⁾ | 0,001 | 89 | 75 |
| | 0,003 | 70 | 18 |
| Sucroselaurat ²⁾ | 0,003 | 86 | 57 |
| | 0,01 | 86 | 13 |

| | | | |
|---|---|---|---|
| 1) Ryoto S1670 | | | |
| 2) Ryoto L1695 | | | |

Die Ergebnisse zeigen, dass die Testsubstanzen die Melaninsynthese in den B16-Melanocyten signifikant inhibieren.

### G) Antibakterielle Wirksamkeit

Die antibakterielle Wirksamkeit der Testsubstanzen wurde mit Hilfe der Diffusionsmethode auf Agar-Platten bzw. der Agar-Verdünnungsmethode untersucht. Dabei wird zunächst ein rundes Filterpapier definierten Durchmessers mit der Testlösung getränkt und dieses dann auf die Oberfläche einer Agarplatte aufgebracht, welche zuvor mit den Testmikroorganismen inokuliert worden war. Anschließend wird nach definierten Zeiten die Größe der Inhibierungszonen bestimmt. Diese Technik erlaubt es insbesondere die MIC (Minimum Inhibitory Concentration) als die niedrigste Testsubstanzkonzentration zu bestimmen, mit der noch eine vollständige Inhibierung der Mikroorganismen erzielt werden kann.

*Agar-Diffusionsmethode.* Das Inokulum wurde unter Verwendung einer frischen Kultur in einer stationären Wachstumsphase (nach ca. 18-24 h) in einer BHI-Lösung (Brain-Heart-InfusionI) hergestellt. Die Bakteriensuspension wurde auf 0,5 MacFarland-Einheiten eingestellt, entsprechend 1,5 10⁸ Kolonienbildende Einheiten (cfu)/ml; anschließend wurde die Suspension mit einer Kochsalzlösung 1/100 verdünnt, um einen Wert von 1,5 10⁶ cfu/ml einzustellen. Anschließend wurde Mueller-Hinton Agar (*Staphylococcus epidermis, Staphylococcus aureus)* und Wilkins-Chalgrens Agar mit 5 Gew.-% Schafsblut *(Propionobakterium acnes)* 15 min bei 121 °C sterilisiert und dann in Petrischalen gegeben. Die Petrischalen wurden mit 2 bis 4 ml der Bakteriensuspensionen versetzt und bei Raumtemperatur getrocknet. Die Filterpapiere wurden mit 20 µl der Testsubstanzen getränkt und auf die Oberfläche der Agarplatten aufgebracht. Die inokulierten Petrischalen wurden 18 bis 24 bei 37 °C inkubiert (die Petrischalen mit Propionibacterium acnes unter anaeroben Bedingungen) und die antimikrobielle Wirksamkeit anschließend durch Bestimmung der Wachstumsinhibierungszone ermittelt. Die Ergebnisse sind in Tabelle 8 zusammengefasst. Angegeben sind ist jeweils der Durchmesser der Inhibierungsflächen in mm.

*Agar Verdünnungsmethode (MIC-Bestimmung).* Wie oben beschrieben wurden verschiedene Agars vorbereitet, mit unterschiedlichen Konzentrationen an Testsubstanzen versetzt, homogenisiert und dann getrocknet. Anschließend erfolgte die Inokulation der Petrischalen mit jeweils 2 µl der Bakteriensuspensionen. Nach dem erneuten Trocknen wurden die Petrischalen bei 37 °C 18 bis 24 h inkubiert. In Tabelle 9 sind die MIC in mg/ml angegeben, d.h. die geringsten Konzentrationen, mit denen noch eine vollständige Inhibierung des Bakterienwachstums erreicht werden kann. Es handelt sich jeweils um den Mittelwert von Doppelbestimmungen.

**Tabelle 8:**

| Inhibierungszonen [mm] | | | |
|---|---|---|---|
| Zuckerester | Staphylococcus aureus | Staphylococcus epidermidis | Propionibacterium acnes |
| Fructosecaprat | 10 | 9 | 14 |
| Glucosecaprylat | 11 | 0 | 12 |
| Glucoselaurat | 14 | 16 | 10 |
| Trehalosecaprat | 10 | 0 | 10 |
| Trehalosepalmitat | 14 | 11 | 0 |
| Trehalosestearat | 12 | 11 | 0 |

**Tabelle 9:**

| MIC [mg/ml] | | | |
|---|---|---|---|
| Zuckerester | Staphylococcus epidermidis | Staphylococcus aureus | Propionibacterium acnes |
| Fructosecaprat | 1,25 | 1,25 | 0,625 |
| Trehalosestearat | 0,625 | >10 | 1,25 |

Die Ergebnisse zeigen, dass die Testsubstanzen über ausgezeichnete antimikrobielle Eigenschaften speziell gegenüber solchen Keimen verfügen, die in die Entstehung von Akne involviert sind.

In den nachfolgenden Tabelle finden sich eine Reihe von Formulierungsbeispielen.

**Tabelle 10**

| Weiße Emulsionen mit Zuckerestern als Emulgatoren (Mengenangaben als Gew.-%) | | | | | |
|---|---|---|---|---|---|
| Phase | Inhaltsstoff | A1 | A2 | A3 | A4 |
| Phase I | Cetearyl Alcohol | 3 | 3 | 3 | 3 |
| | Cetearyl Isononanoate | 15 | 15 | 15 | 15 |
| | Caprylic Capric Triglycerides | 6 | 6 | 6 | 6 |
| | Fructosepalmitat | 3 | - | - | - |
| | Trehalosepalmitat | - | 3 | - | - |
| | Trehalosestearat | - | - | 3 | - |
| | Sucrosestearat ¹⁾ | - | - | - | 3 |
| Phase 2 | Wasser | ad 100 | | | |
| | Elestab® 388 | 2,5 | 2,5 | 2,5 | 2,5 |
| | Keltrol® T | 0,2 | 0,2 | 0,2 | 0,2 |
| | NaOH 1N | 0,2 | 0,2 | 0,2 | - |
| Phase 3 | Citric acid (10 % b.w.) | - | 0,1 | 0,1 | - |

| | | | | | |
|---|---|---|---|---|---|
| 1) Sisterna SP50C | | | | | |

Zur Herstellung der Emulsionen wurde die Phase 1 bei 75 °C vorgelegt und die auf die gleiche Temperatur erhitzte Phase 2 unter starkem Rühren hinzugegeben. Nach Abkühlen auf Raumtemperatur wurde die Phase 3 eingerührt. Die Emulsionen wiesen in 5 Gew.-%iger Verdünnung einen pH-Wert von 6,2 bis 7,8 sowie eine Viskosität nach Brookfield von 120 bis 600 ps auf.

**Tabelle 11**

| Weiße Emulsionen mit Zuckerestern als Co-Emulgatoren (Mengenangaben als Gew.-%) | | | | | | | |
|---|---|---|---|---|---|---|---|
| Phase | Inhaltsstoff | B1 | B2 | B3 | B4 | B5 | B6 |
| Phase 1 | Cetearyl Alcohol (and) Ceteareth 20 | 5 | 5 | 5 | 5 | 5 | 5 |
| | Cetearyl Alcohol | 3 | 3 | 3 | 3 | 3 | 3 |
| | Cetearyl Isononanoate | 15 | 15 | 15 | 15 | 15 | 15 |
| | Caprylic Capric Triglycerides | 3 | 3 | 3 | 3 | 3 | 3 |
| | Fructosecaprat | 3 | - | - | - | - | - |
| | Glucosecaprylat | - | 3 | - | - | - | - |
| | Glucoselaurat | - | - | 3 | - | - | - |
| | Trehalosecaprat | - | - | - | 3 | - | - |
| | Trehaloselaurat | - | - | - | - | 3 | - |
| | Sucroselaurat ¹⁾ | - | - | - | - | - | 3 |
| Phase 2 | Wasser | ad 100 | | | | | |
| | Elestab® 388 | 2,5 | 2,5 | 2,5 | 2,5 | 2,5 | 2,5 |
| | Keltrol® T | 0,2 | 0,2 | 0,2 | 0,2 | 0,2 | 0,2 |
| | NaOH 1N | - | 0,2 | 0,2 | 0,1 | 0,1 | - |

| | | | | | | | |
|---|---|---|---|---|---|---|---|
| 1) Ryoto L595 | | | | | | | |

Zur Herstellung der Emulsionen wurde die Phase 1 bei 75 °C vorgelegt und die auf die gleiche Temperatur erhitzte Phase 2 unter starkem Rühren hinzugegeben. Die Emulsionen wiesen in 5 Gew.-%iger Verdünnung einen pH-Wert von 6,2 bis 7,2 sowie eine Viskosität nach Brookfield von 200 bis 600 ps auf.

**Tabelle 12**

| Schäumende Zubereitungen (Mengenangaben als Gew.-%) | | | | | | | |
|---|---|---|---|---|---|---|---|
| Inhaltsstoff | | C1 | C2 | C3 | C4 | C5 | C6 |
| Phase 1 | Fructosecaprat | 2,5 | - | - | - | - | - |
| | Glucosecaprylate | | 2,5 | - | - | - | - |
| | Glucoselaurat | - | - | 2,5 | - | - | - |
| | Trehalosecaprat | - | - | - | 2,5 | - | - |
| | Trehaloselaurat | - | - | - | - | 2,5 | - |
| | Sucroselaurat ¹⁾ | - | - | - | - | - | 6.25 |
| | Elestab® 388 | 2,5 | 2,5 | 2,5 | 2,5 | 2,5 | 2,5 |
| | Wasser | ad 100 | | | | | |
| Phase 2 | Sodium Laureth Sulfate | 30 | 30 | 30 | 30 | 30 | 30 |
| | Cocamidopropyl Betaine | 6 | 6 | 6 | 6 | 6 | 6 |
| | Sodium chloride | 0,5 | 0,5 | 0,5 | 0,5 | 0,5 | 0,5 |

| | | | | | | | |
|---|---|---|---|---|---|---|---|
| 1) Sisterna L 70C | | | | | | | |

Zur Herstellung der Zubereitungen wurde die Phase 1 bei 75 °C vorgelegt, zunächst das Sodium Laureth Sulfate eingerührt und dann nach Abkühlen auf Raumtemperatur die übrigen Bestandteile der Phase II hinzugegeben. Die Emulsionen wiesen in 5 Gew.-%iger Verdünnung einen pH-Wert von 6 bis 7 sowie eine Viskosität nach Brookfield von 200 bis 2500 cps auf.

## Patentansprüche

1. Verwendung von Zuckerestern als Wirkstoffe zur Herstellung von kosmetischen und/oder pharmazeutischen Zubereitungen.

2. Verwendung von Zuckerestern als anti-inflammatorische Wirkstoffe.

3. Verwendung von Zuckerestern als Wirkstoffe zum Schutz von Haut und Haaren gegen die Einwirkung von UV-Strahlen

4. Verwendung von Zuckerestern als Wirkstoffe zum Schutz von Haut und Haaren gegen Umweltgifte und oxidativen Stress.

5. Verwendung von Zuckerestern als Anti-Ageingmittel.

6. Verwendung von Zuckerestern als Anti-Protease-Wirkstoffe.

7. Verwendung von Zuckerestern als Wirkstoffe zur Inhibierung der Melaninsynthese in Haar- und Hautzellen.

8. Verwendung von Zuckerestern als Anti-Akne-Wirkstoffe.

9. Verwendung nach mindestens einem der Ansprüche 1 bis 8, **dadurch gekennzeichnet, dass** man Zuckerester einsetzt, die sich von Mono-, Di und/oder Oligosacchariden ableiten.

10. Verwendung nach Anspruch 9, **dadurch gekennzeichnet, dass** man Saccharide einsetzt, die ausgewählt sind aus der Gruppe, die gebildet wird von Glucose, Methylglucose, Mannose, Galactose, Rhamnose, Fucose, Ribose, Desoxyribose, Arabinose, Xylose, Sucrose, Maltose, Isomaltose, Cellobiose, Melibiose, Gentobiose, Lactose und Trehalose.

11. Verwendung nach mindestens einem der Ansprüche 1 bis 10, **dadurch gekennzeichnet, dass** man Zuckerester einsetzt, die sich von Fettsäuren der Formel (I) ableiten,
R¹CO-OH (I)
in der R¹CO für einen linearen oder verzweigten, gesättigten oder ungesättigten Acyl oder Hydroxyacylrest mit 6 bis 22 Kohlenstoffatomen und 0 und/oder 1 bis 3 Doppelbindungen steht.

12. Verwendung von Zuckerestern nach mindestens einem der Ansprüche 1 bis 11, **dadurch gekennzeichnet, dass** man Zuckerester einsetzt, die sich von Dicarbonsäuren mit 22 bis 22 Kohlenstoffatomen ableiten.

13. Verwendung von Zuckerestern nach mindestens einem der Ansprüche 1 bis 12, **dadurch gekennzeichnet, dass** man Zuckerester einsetzt, die einen Veresterungsgrad von durchschnittlich 1 bis 6 aufweisen.

14. Verwendung von Zuckerestern nach mindestens einem der Ansprüche 1 bis 13, **dadurch gekennzeichnet, dass** man die Zuckerester in Mengen von 0,0001 bis 10 Gew.-% - bezogen auf die Mittel - einsetzt.
